Europäisches Patentamt

European Patent Office

Office européen des brevets

(11) Publication number: **0 546 213 A1**

## EUROPEAN PATENT APPLICATION

(21) Application number: **91121267.8**

(22) Date of filing: **11.12.91**

(51) Int. Cl.⁵: **C07K 15/08**, A61K 37/02, C12P 21/02, C12N 5/00

---

The applicant has subsequently filed a sequence listing and declared, that it includes no new matter.

(43) Date of publication of application:
**16.06.93 Bulletin 93/24**

(84) Designated Contracting States:
**DE FR GB IT NL SE**

(71) Applicant: **TORAY INDUSTRIES, INC.**
**2-1, Nihonbashi Muromachi 2-chome**
**Chuo-ku**
**Tokyo 103(JP)**

(72) Inventor: **Yamada, Katsushige**
**10-10, Shikae-cho 1-chome**
**Hoya-shi, Tokyo 202(JP)**
Inventor: **Natori, Shunji**
**2208-19, Fukawa, Tone-machi**
**Kitasoma-gun, Ibaraki 270-12(JP)**

(74) Representative: **Kador & Partner**
**Corneliusstrasse 15**
**W-8000 München 5 (DE)**

---

(54) **Biologically active polypeptide and antibacterial agent.**

(57) The present invention relates to a biologically active polypeptide and an antibacterial agent containing the same. The biologically active polypeptide is having the following amino acid sequence:

$H_2$N-Ala-Thr-Cys-Asp-Leu-Leu-Ser-Gly-Ile-Gly-Val-Gln-His-Ser-Ala-Cys-Ala-Leu-His-Cys-Val-Phe-Arg-Gly-Asn-Arg-Gly-Gly-Tyr-Cys-Thr-Gly-Lys-Gly-Ile-Cys-Val-Cys-Arg-Asn-COOH.

This polypeptide is produced by cultured cells originated from embryo of Sarcophaga peregrina and is used in a pharmaceutical composition for treating or preventing infectious diseases.

EP 0 546 213 A1

Fig.1

BACKGROUND OF THE INVENTION

I. Field of the Invention

This invention relates to a biologically active polypeptide and an antibacterial agent containing the same.

II. Description of the Related Art

It is known that when a vaccine or the like is inoculated to invertebrates such as insects, antibacterial substances emerge in the body fluid thereof (European Journal of Biochemistry, vol. 106, p.7 (1980)). The characteristics of these substances as well as the mechanism of the antibacterial action have not been sufficiently clarified.

It has also been shown that there are polypeptides with strong antibacterial activities and wide antibacterial spectra in seminal fluids (Nature, vol. 279, p.725 (1979)) or in sera (Biochemistry, vol. 20, p.5973 (1981)) of mammals. Thus, the antibacterial proteins in animals and insects are now drawing attention.

It is known that a polypeptide with a strong antibacterial activity against Escherichia coli or Bacillus subtilis appears in the body fluid of flesh fly (Sarcophaga peregrina) when the larva of the insect is injured (Japanese laid-open patent application (Kokai) Nos. 59-13730 and 61-122299).

Further, it was found that a polypeptide having an antibacterial activity exists in the culture supernatant of the cultured cells originated from the embryo of Sarcophaga peregrina. The antibacterial polypeptide has been purified and the characteristics and structure have been identified (European patent publication No. 0 280 859).

Since the antibacterial polypeptide originated from Sarcophaga peregrina is, as in the case of other insects, specifically induced when the larva of the insect is injured, the polypeptide is thought to be a defensive substance for preventing the bacterial infection. Further, since a plurality of antibacterial proteins exist, it is thought that these proteins mutually interact so as to constitute a defensive system in Sarcophaga peregrina.

The antibacterial polypeptides originated from Sarcophaga peregrina have wide antibacterial spectra and strong antibacterial activities. Further, they are proteins, so that they may be edible. Thus, they are expected as edible antibacterial agents.

However, a novel antibacterial agent with higher activity against undesirable pathogens and other microorganisms is continuously demanded, so that the discovery of a novel antibacterial polypeptide in the antibacterial potypeptides originated from Sarcophaga peregrina is desired.

SUMMARY OF THE INVENTION

Accordingly, an object of the present invention is to provide a novel biologically active polypeptide with antibacterial activity.

Another object of the present invention is to provide an antibacterial agent with strong antibacterial activity and wide antibacterial spectrum.

The present inventors intensively studied to discover a novel biologically active polypeptide with strong antibacterial activity and wide antibacterial spectrum from the culture supernatant of cultured cells of Sarcophaga peregrina embryo, so as to complete the present invention.

That is, the present invention provides a biologically active polypeptide having the amino acid sequence of the following formula [I]:

H₂N-Ala-Thr-Cys-Asp-Leu-Leu-Ser-Gly-Ile-Gly-Val-Gln-His-
Ser-Ala-Cys-Ala-Leu-His-Cys-Val-Phe-Arg-Gly-Asn-Arg-Gly-
Gly-Tyr-Cys-Thr-Gly-Lys-Gly-Ile-Cys-Val-Cys-Arg-Asn-COOH

[I]

3

The present invention also provides an antibacterial agent containing the biologically active polypeptide of the present invention as an effective ingredient.

The present invention still further provides a pharmaceutical composition for treating or preventing infectious diseases comprising an effective amount of the antibacterial polypeptide of the present invention in a pharmaceutically acceptable carrier.

By the present invention, a novel biologically active polypeptide having a strong antibacterial activity and a wide antibacterial spectrum, which has substantially no toxicity, was provided. By virtue of the strong antibacterial activity and of the substantially no toxicity, the antibacterial polypeptide of the present invention may find a wide variety of uses including antibacterial agents and food additives. Especially, since the antibacterial polypeptide of the present invention is a protein and has a good thermal stability, it is expected that the antibacterial polypeptide is suited as an antibacterial food additive to be added to a processed food for which a heat treatment is performed in the processing of the food.

BRIEF DESCRIPTION OF THE DRAWINGS

Fig. 1 is a chromatogram showing the elution pattern of the antibacterial polypeptide of the present invention on a CM cellulose column chromatography during the purification process thereof;

Fig. 2 is a chromatogram showing the elution pattern of the antibacterial polypeptide of the present invention on Sephadex G-50 column chromatography during the purification process thereof; and

Fig. 3 is a chromatogram showing the elution pattern of the antibacterial polypeptide of the present invention on HPLC which is the final step of the purification process thereof.

DETAILED DESCRIPTION OF THE PREFERRED EMBODIMENTS

As stated above, the antibacterial polypeptide of the present invention has the amino acid sequence represented by the above-described formula [I]. In the formula [I], "$H_2N$-" means that the alanine is the amino terminal amino acid residue and "-COOH" means that the asparagine is the carboxyl terminal amino acid residue.

The antibacterial polypeptide of the present invention was prepared from the culture supernatant of the cultured cells originated from embryo of Sarcophaga peregrina. The purification process of the antibacterial polypeptide from the culture supernatant is detailed in the actual working example hereinbelow described. Thus, those skilled in the art can obtain the antibacterial polypeptide of the present invention by following the procedure described in the example. Alternatively, since the structure of the antibacterial polypeptide of the present invention was determined as shown in the formula [I], the antibacterial polypeptide may be synthesized by using a commercially available peptide synthesizer. Still alternatively, since the structure of antibacterial polypeptide was determined, a DNA encoding the antibacterial polypeptide of the present invention may be easily prepared and the antibacterial polypeptide may be produced by a genetic engineering process employing the DNA and a commercially available expression vector.

The antibacterial polypeptide of the present invention may be used as an antibacterial agent by itself or by combining with other components in the form of liquid or solid.

More particularly, the antibacterial polypeptide of the present invention may be added as a preservative or a disinfectant to washing solutions of contact lenses, ointments, soaps, shampoos, pharmaceuticals, foods, feeds, pet foods, tooth pastes and the like.

The antibacterial polypeptide of the present invention may also be used for treating or preventing bacterial infectious diseases. In this case, the antibacterial polypeptide may be administered orally or parenterally.

When the antibacterial polypeptide is used as a pharmaceutical for treating or preventing bacterial infectious diseases, the antibacterial polypeptide may be formulated in the form of tablet, sugar-coated tablet, pill, capsule, powder, troche, solution, suppository, injection or the like, together with one or more pharmaceutically acceptable excipients. Examples of the pharmaceutically acceptable excipients include lactose, sucrose, glucose, sorbitol, mannitol, potato starch, amylopectin, other starches, cellulose derivatives such as carboxymethyl cellulose and hydroxyethyl cellulose, gelatin, magnesium stearate, polyvinyl alcohols, calcium stearate, polyethylene glycol waxes, Arabian gum, talc, titanium dioxide, plant oils such as olive oil, peanut oil and sesame oil, paraffin oil, neutral fat bases, ethanol, propylene glycol, physiological saline, sterilized water, glycerin, coloring agents, seasonings, thickening agents, stabilizers, isotonic agents, buffering agents and the like.

The antibacterial agent or the antibacterial pharmaceutical composition may preferably contain the antibacterial polypeptide of the present invention in the amount of 0.001 - 85% by weight, more preferably 0.005 - 60% by weight.

The pharmaceutical containing the antibacterial polypeptide of the present invention as an effective ingredient may be administered with a dose of, for example, 1 - 500 mg/day, more preferably 1 - 50 mg/day in terms of the effective ingredient for an adult, although the dose is determined mainly based on the state of the patient.

Since the antibacterial agent according to the present invention is a protein, the antibacterial agent is thought to have substantially no toxicity at least when it is orally administered. Therefore, the antibacterial agent of the present invention is especially useful as an additive to be added to drugs for human and animals, foods and feeds. For example, by adding the antibacterial polypeptide of the present invention to foods such as chewing gums or to tooth pastes, disinfection in the mouth or treatment or prevention of dental carries and periodontal diseases may be attained. Further, in the cultivation of fishes, by adding the antibacterial polypeptide of the present invention to the feeds of the fishes, protection of the fishes from infectious diseases may be attained.

Thus, the antibacterial polypeptide of the present invention may be used as a preservative, pharmaceutical or food additive containing the antibacterial polypeptide as an effective ingredient.

The present invention will now be described by way of examples thereof. It should be noted that the examples are presented for the illustration purpose only and should not be interpreted in any restrictive way.

Example 1

Preparation of Antibacterial Polypeptide

(a) Cell Culture

In aseptical filtrate of the medium disclosed by Mitsuhashi et al. (containing 7g/l of sodium chloride, 0.2 g/l of potassium chloride, 0.2 g/l of calcium chloride dihydrate, 0.1 g/l of magnesium chloride hexahydrate, 0.2 g/l of sodium dihydrogen phosphate, 0.12 g/l of sodium hydrogen carbonate, 4 g/l of glucose, 6.5 g/l of lactoalbumin hydrolysate and 5 g/l of yeastlate, pH 6.5), cells originated from Sarcophaga peregrina embryo (NIH sape-4 cell) were inoculated to the medium to a population density of $1 \times 10^6$ cells/ml. The cells were cultured at 25°C for 7 - 10 days. When the population density reached about $2 \times 10^7$ cells/ml, the cells were subcultured. The subculture was centrifuged and the supernatant was recovered.

(b) Purification of Antibacterial Polypeptide

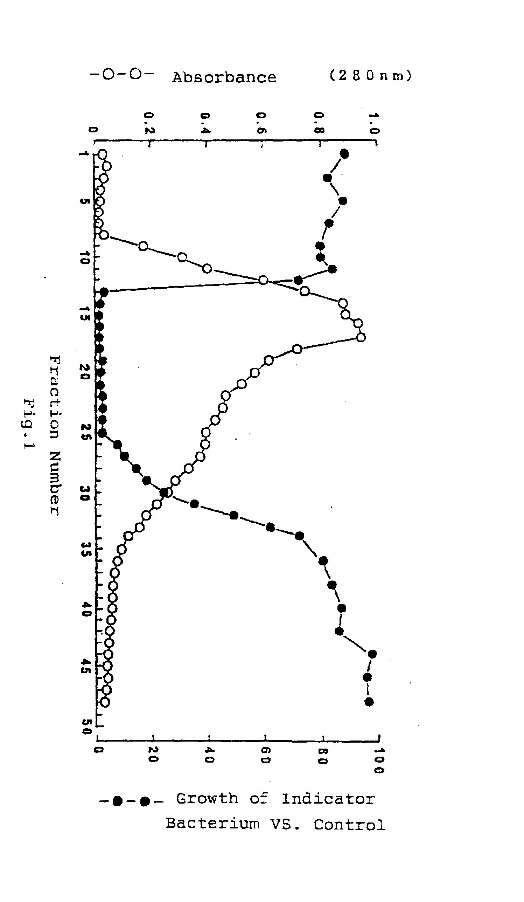

To 900 ml of the culture supernatant obtained in (a), 2700 ml of phosphate buffer (pH 6.0) was added and the pH and salt concentration were adjusted. The resulting solution was passed through a CM cellulose column (3.0 x 9.0 cm) and was washed with the same phosphate buffer. The same buffer but containing 520 mM NaCl was passed through the column and the eluted solution was fractioned into 4 ml each. The absorbance at 280 nm and the antibacterial activity of each fraction were measured. The antibacterial activity was measured using Staphylococcus aureus (IFO 12732) according to the method described in Journal of Biochemistry, vol. 211, pp.724-734 (1983)). The results are shown in Fig. 1.

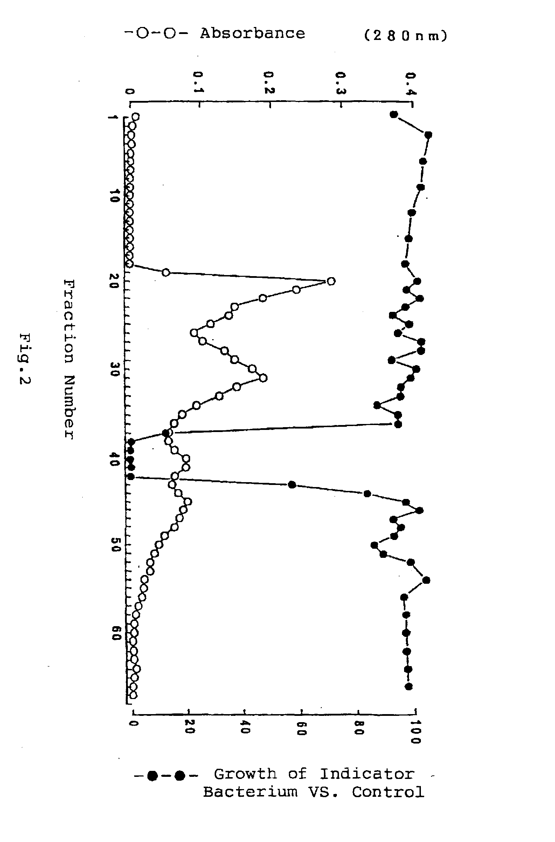

The fractions having antibacterial activities were combined and heated at 100°C for 10 minutes. The formed precipitates were removed by centrifugation and the obtained supernatant was concentrated by ultrafiltration. The thus obtained concentrate was passed through Sephadex G-50 column (1.5 x 120 cm, commercially available from Pharmacia), and eluted with a phosphate buffer containing 130 mM NaCl. The eluted solution was fractioned into 4 ml each and the absorbance at 280 nm and the antibacterial activity of each fraction were measured as mentioned above. The results are shown in Fig. 2.

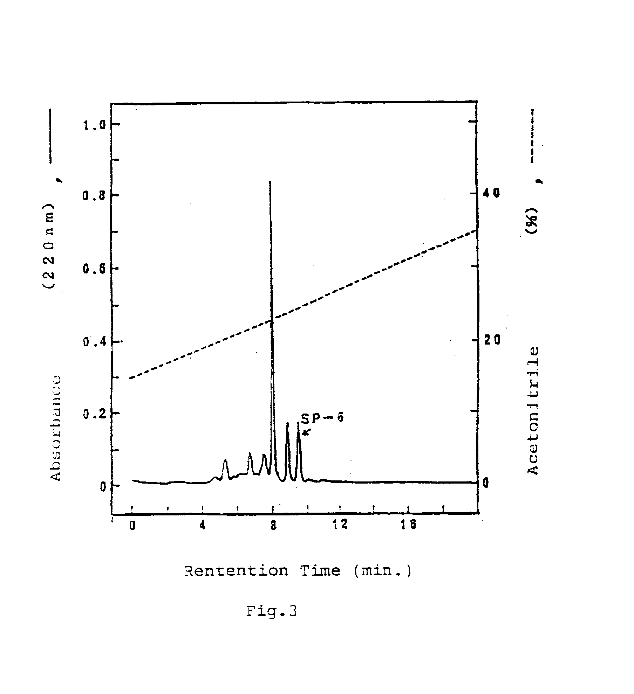

The fractions having antibacterial activities were combined and subjected to HPLC reverse phase chromatography. The results of the HPLC are shown in Fig. 3. By recovering the peak fraction in the chromatogram shown in Fig. 3, the desired antibacterial polypeptide SP-6 was obtained. The conditions of the HPLC were as follows:

Column: Synchropak RP-P (commercially available from SynChron, Inc.)
Eluent:
Solution A: Aqueous 0.05 vol% trifluoroacetic acid
Solution B: 0.05.vol% trifluoroacetic acid in acetonitrile
Gradient: linear gradient of 15% to 40% of solution B with respect to the total of solutions A and B
Flow Rate: 2 ml/min.

The total protein, total activity, specific activity, and activity yield after each step during the above-described purification process are summarized in Table 1 below.

Table 1

| Purification Step | Total Protein (mg) | Total Activity (unit) | Specific Activity (unit/mg) | Activity Yield (%) |
|---|---|---|---|---|
| Culture Supernatant | 3258 | 6675 | 2 | 100 |
| CM-Cellulose | 26.8 | 4247 | 159 | 63.6 |
| Heat Treatment | 18.3 | 3406 | 186 | 51.0 |
| Concentration | 15.8 | 2912 | 184 | 43.6 |
| Sephadex G-50 | 5.3 | 1986 | 375 | 29.8 |
| HPLC | 0.08 | 207 | 2583 | 3.7 |

Example 2

Characterization of SP-6

(a) Amino Acid Sequence of SP-6

The amino acid sequence of the thus obtained SP-6 was determined by subjecting it to Edman degradation using a commercially available gas phase sequencer (Type 477A, commercially available from Applied Biosystems, Inc.) and identifying the obtained PTH-amino acids using PTH-analyzer (Type 120A, commercially available from Applied Biosystems, Inc). The determined amino acid sequence was as follows:

$$H_2N-Ala-Thr-Cys-Asp-Leu-Leu-Ser-Gly-Ile-Gly-Val-Gln-His-$$

$$Ser-Ala-Cys-Ala-Leu-His-Cys-Val-Phe-Arg-Gly-Asn-Arg-Gly-$$

$$Gly-Tyr-Cys-Thr-Gly-Lys-Gly-Ile-Cys-Val-Cys-Arg-Asn-COOH$$

(b) Amino Acid Composition of SP-6

SP-6 was hydrolyzed with 6N hydrochloric acid containing 4 wt% thioglycollic acid in a vacuum tube at 110°C for 22 hours. The composition of the amino acids in the resulting hydrolysate was determined using a commercially available amino acid analyzer (Type 835, commercially available from Hitachi Ltd). The molar ratio of the amino acids is shown in Table 2 below.

6

Table 2

| | |
|---|---|
| Aspartic Acid (Asp) + Asparagine (Asn) | 3.07 (3) |
| Threonine (Thr) | 1.75 (2) |
| Serine (Ser) | 1.49 (2) |
| Glutamic Acid (Glu) + Glutamine (Gln) | 1.23 (1) |
| Glycine (Gly) | 7.06 (7) |
| Alanine (Ala) | 3.29 (3) |
| Valine (Val) | 3.03 (3) |
| Isoleucine (Ile) | 1.78 (2) |
| Leucine (Leu) | 3.14 (3) |
| Tyrosine (Tyr) | 1.00 (1) |
| Phenylalanine (Phe) | 1.00 (1) |
| Lysine (Lys) | 1.10 (1) |
| Histidine (His) | 1.95 (2) |
| Arginine (Arg) | 2.97 (3) |
| 1/2 Cystein | 5.84 (6) |
| (The molar ratio is expressed taking the average of tyrosine and phenylalanine as 1.0. The values in the parentheses indicate the number of amino acid residues counted from the above-described amino acid sequence). | |

The results shown in Table 2 are consistent with the above-described amino acid sequence.

(c) Antibacterial Activities

According to the method described in Journal of Biochemistry vol. 211, pp.727-734, 1983, antibacterial activities against various bacteria were determined. The results are shown in Table 3 below.

Table 3

| Test Bacteria | Minimal Growth-Inhibiting Concentration (μg/ml) |
|---|---|
| Staphylococcus aureus IFO 12732 | 2.0 |
| Staphylococcus aureus SR 3626 | 4.6 |
| Staphylococcus aureus SR 1550 | 6.6 |
| Staphylococcus aureus SR 1587 | 0.9 |
| Staphylococcus aureus SR 3636 | 5.4 |
| Streptococcus mutans 6715 | 4.5 |
| Streptococcus sanguis B220 | 5.0 |
| Streptococcus sanguis ST3 | 1.8 |
| Streptococcus salivarius HHT | 2.1 |
| Escherichia coli 594 | >10 |
| Bacillus circulans IFO 3967 | 0.25 |
| Bacillus megaterium IAM 1161 | 0.25 |
| Proteus mirabills IFO 3849 | >10 |
| Klebssiela pneumoniae ATCC 21694 | >10 |

Further, the antibacterial activities of SP-6 and KM-2 (the antibacterial polypeptide described in European patent publication No. 280,859) were determined. The results are shown in Table 4 below.

7

Table 4

| Test Bacteria | 50% Growth-Inhibiting Concentration* (μg/ml) | |
|---|---|---|
| | KM-2 | SP-6 |
| Corynebacterium glutamicum ATCC 13059 | 0.24 | 0.17 |
| Streptococcus mutans OZM 176 | 18.5 | 6.90 |
| Streptococcus mutans MT 8148 | 15.0 | 6.1 |
| Streptococcus mutans 6715 | 4.5 | 2.6 |
| Streptococcus sanguis ST3 | 1.5 | 0.9 |
| Streptococcus salivarius HHT | 2.6 | 0.7 |

*) 50% growth-inhibiting concentration is shown as the concentration causing 50% growth inhibition compared with control.

# SEQUENCE LISTING

**SEQUENCE LENGTH: 40**

**SEQUENCE TYPE: amino acid**

**TOPOLOGY: linear**

**MOLECULE TYPE: peptide**

**SEQUENCE DESCRIPTION:**

Ala Thr Cys Asp Leu Leu Ser Gly Ile Gly Val Gln His
                5                   10

Ser Ala Cys Ala Leu His Cys Val Phe Arg Gly Asn Arg Gly
     15                  20                  25

Gly Tyr Cys Thr Gly Lys Gly Ile Cys Val Cys Arg Asn
         30                  35

## Claims

1. A biologically active polypeptide having the amino acid sequence of the formula:

$$H_2N-Ala-Thr-Cys-Asp-Leu-Leu-Ser-Gly-Ile-Gly-Val-Gln-His-$$

$$Ser-Ala-Cys-Ala-Leu-His-Cys-Val-Phe-Arg-Gly-Asn-Arg-Gly-$$

$$Gly-Tyr-Cys-Thr-Gly-Lys-Gly-Ile-Cys-Val-Cys-Arg-Asn-COOH.$$

2. The biologically active polypeptide of claim 1, which is produced by cultured cells originated from embryo of Sarcophaga peregrina.

3. An antibacterial agent comprising effective amount of said biologically active polypeptide of claim 1 as an effective ingredient.

4. A pharmaceutical composition for treating or preventing infectious diseases comprising an effective amount of the antibacterial polypeptide of claim 1 in a pharmaceutically acceptable carrier.

Fig. 1

Fig. 2

Fig.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 280 859 (SANWA KAGAKU KENKYUSHO CO.) 7 September 1988 <br> * claims 1-3 * <br> --- | 1-4 | C07K15/08 <br> A61K37/02 <br> C12P21/02 <br> C12N5/00 |
| A | EP-A-0 349 451 (CNRS) 3 January 1990 <br> * claims 1-9 * <br> --- | 1,3,4 | |
| A | MOL. CELL. BIOL. <br> vol. 10, no. 12, December 1990, WASHINGTON, US <br> pages 6114 - 6122; <br> A. KANAI & S. NATORI: 'Analysis of a Gene Cluster for Sarcotoxin II, a Group of Antibacterial Proteins of Sarcophaga peregrina' <br> * page 6114, left column, paragraph 3 * <br> * page 6121, left column, paragraph 3 * <br><br> ----- | 1 | |
| | | | TECHNICAL FIELDS SEARCHED (Int. Cl.5) <br><br> C07K |

The present search report has been drawn up for all claims

| Place of search | Date of completion of the search | Examiner |
|---|---|---|
| THE HAGUE | 20 JULY 1992 | THIELE U.H.-C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)